# EUROPEAN PATENT APPLICATION

(11) **EP 1 281 765 A1**
(43) Date of publication of application: **05.02.2003**
(21) Application number: 00955091.4
(22) Date of filing: 29.08.2000
(51) Int. Cl.: C12N 15/85, C12N 5/10, A01K 67/027

(54) **ANTISENSE TYPE GENE TRAP VECTOR**

(30) Priority: 11.05.2000 JP 2000138938
(71) Applicant: Japan Science and Technology Corporation, Kawaguchi-shi, Saitama-ken 332-0012 (JP)
(72) Inventor: TANIGUCHI, Masaru, Chiba-shi, Chiba 263-0043 (JP); KARASAWA, Mika, Niiza-shi, Saitama 352-0014 (JP)
(74) Representative: Harding, Charles Thomas
(86) International application number: JP0005824
(87) International publication number: WO01085973

(57) **Abstract**

To provide the followings that are applicable for in vitro cell-differentiation systems other than ES cells: cells whose function of expressing normal genes (wild type genes) is deficient or suppressed in the alleles corresponding to trapped genes, and the preparation method for the cells; a gene-trap method using the cells; and antisense promoter insertion vector for use in preparation of the cells. Not only trap-clone is efficiently obtained by introducing a gene-trap vector into the cells, but antisense RNA to the trapped genes are forcibly transcribed and the transcipts from wild type genes are disrupted by introducing, in inverse direction to the endogenous gene, antisense promoter insertion vector which is capable of inserting a strong promoter in a certain position of the mutant locus where the gene-trap vector of the trap-clone is inserted in.

SEQUENCE LISTING

<110> JAPAN SCIENCE AND TECHNOLOGY CORPORATION

<120> Antisense Gene Trap Vector

<130> A031-26PCT

<140>
<141>

<150> JP 2000/138938
<151> 2000-05-12

<160> 6

<170> PatentIn Ver. 2.1

<210> 1
<211> 34
<212> DNA
<213> Bacteriophage P1

<300>
<301> Araki, K
      Araki, M
      Yamamura, K
<302> Targeted integration of DNA using mutant lox sites in
      embryonic stem cells
<303> Nucleic Acids Res.
<304> 25
<305> 4
<306> 868-872
<307> 1997

<400> 1
ataacttcgt atagcataca ttatacgaag ttat                                34


<210> 2
<211> 34
<212> DNA
<213> Artificial Sequence

<220>
<223> Description of Artificial Sequence:lox71 sequence

<300>
<301> Araki, K
      Araki, M
      Yamamura, K
<302> Targeted integration of DNA using mutant lox sites in
      embryonic stem cells
<303> Nucleic Acids Res.
<304> 25
<305> 4
<306> 868-872
<307> 1997

<400> 2
taccgttcgt atagcataca ttatacgaag ttat                                34

<210> 3
<211> 34
<212> DNA

<213> Artificial Sequence

<220>
<223> Description of Artificial Sequence:lox66 sequence

<300>
<301> Araki, K
      Araki, M
      Yamamura, K
<302> Targeted integration of DNA using mutant lox sites in
      embryonic stem cells
<303> Nucleic Acids Res.
<304> 25
<305> 4
<306> 868-872
<307> 1997

<400> 3
ataacttcgt atagcataca ttatacgaac ggta                                34


<210> 4
<211> 34
<212> DNA
<213> Saccharomyces cerevisiae

<300>
<301> Ringrose, L
      Lounnas, V
      Ehrlich, L
      Buchholz, F
      Wade, R
      Stewart, A. F.
<302> Comparative Kinetic Analysis of FLP and Cre
      Recombinases: Mathematical Models for DNA Binding and
      Recombination
<303> J. Mol. Biol.
<304> 284
<306> 363-384
<307> 1998

<400> 4
gaagttccta ttctctagaa agtataggaa cttc                                34


<210> 5
<211> 34
<212> DNA
<213> Artificial Sequence

<220>
<223> Description of Artificial Sequence:FRTM sequence

<300>
<301> Ringrose, L
      Lounnas, V
      Ehrlich, L
      Buchholz, F
      Wade, R
      Stewart, A. F.
<302> Comparative Kinetic Analysis of FLP and Cre
      Recombinases: Mathematical Models for DNA Binding and
      Recombination
<303> J. Mol. Biol.
<304> 284
<306> 363-384
<307> 1998

<400> 5
gaagttccta tactttctag agaataaacc tctc                                34

<210> 6
<211> 5054
<212> DNA
<213> Artificial Sequence

<220>
<223> Description of Artificial Sequence:Gene Trap
      Vector

<400> 6
ggtaccctat tggagtcctt caaggaaaca aacttggcct caccaggcct cagccttggc 60
tcctcctggg aactctactg cccttgggat cccgctcgac ggtatcgata agcttgatgg 120
ggatccggaa cccttaatat aacttcgtat aatgtatgct atacgaacgg tataggtccc 180
tcgacctgca gcccgatccc ctagtttgtg ataggccttt tagctacatc tgccaatcca 240
tctcattttc acacacacac acaccacttt ccttctggtc agtgggcaca tgtccagccn 300
nncccaacac ttgtatggcc ttggcggggt catccccccc ccacccccag tatctgcaac 360
ctcaagctag cttgggtgcg ttggttgtgg ataagtagct agactccagc aaccagtaac 420
ctctgccctt tctcctccat gacaaccagg tcccaggtcc cgaaaaccaa agaagaagaa 480
ccctaacaaa gaggacaagc ggcctcgcac agccttcact gctgagcagc tccagaggct 540
caaggctgag tttcagacca acaggtacgg ccgctctaga ggaattccgc ccctctccct 600
cccccccccc taacgttact ggccgaagcc gcttggaata aggccggtgt gcgtttgtct 660
atatgttatt ttccaccata ttgccgtctt ttggcaatgt gagggcccgg aaacctggcc 720
ctgtcttctt gacgagcatt cctaggggtc tttcccctct cgccaaagga atgcaaggtc 780
tgttgaatgt cgtgaaggaa gcagttcctc tggaagcttc ttgaagacaa acaacgtctg 840
tagcgaccct ttgcaggcag cggaaccccc cacctggcga caggtgcctc tgcggccaaa 900
agccacgtgt ataagataca cctgcaaagg cggcacaacc ccagtgccac gttgtgagtt 960
ggatagttgt ggaaagagtc aaatggctct cctcaagcgt attcaacaag gggctgaagg 1020
atgcccagaa ggtaccccat tgtatgggat ctgatctggg gcctcggtgc acatgcttta 1080
catgtgttta gtcgaggtta aaaaacgtct aggccccccg aaccacgggg acgtggtttt 1140
cctttgaaaa acacgatgat aagcttgcca caaccatgga tcccgtcgtt ttacaacgtc 1200
gtgactggga aaaccctggc gttacccaac ttaatcgcct tgcagcacat ccccctttcg 1260
ccagctggcg taatagcgaa gaggcccgca ccgatcgccc ttcccaacag ttgcgcagcc 1320
tgaatggcga atggcgcttt gcctggtttc cggcaccaga agcggtgccg gaaagctggc 1380
tggagtgcga tcttcctgag gccgatactg tcgtcgtccc ctcaaactgg cagatgcacg 1440
gttacgatgc gcccatctac accaacgtaa cctatcccat tacggtcaat ccgccgtttg 1500
ttcccacgga gaatccgacg ggttgttact cgctcacatt taatgttgat gaaagctggc 1560
tacaggaagg ccagacgcga attatttttg atggcgttaa ctcggcgttt catctgtggt 1620
gcaacgggcg ctgggtcggt tacggccagg acagtcgttt gccgtctgaa tttgacctga 1680
gcgcattttt acgcgccgga gaaaaccgcc tcgcggtgat ggtgctgcgt tggagtgacg 1740
gcagttatct ggaagatcag gatatgtggc ggatgagcgg cattttccgt gacgtctcgt 1800
tgctgcataa accgactaca caaatcagcg atttccatgt tgccactcgc tttaatgatg 1860
atttcagccg cgctgtactg gaggctgaag ttcagatgtg cggcgagttg cgtgactacc 1920
tacgggtaac agtttcttta tggcagggtg aaacgcaggt cgccagcggc accgcgcctt 1980
tcggcggtga aattatcgat gagcgtggtg gttatgccga tcgcgtcaca ctacgtctga 2040
acgtcgaaaa cccgaaactg tggagcgccg aaatcccgaa tctctatcgt gcggtggttg 2100
aactgcacac cgccgacggc acgctgattg aagcagaagc ctgcgatgtc ggtttccgcg 2160
aggtgcggat tgaaaatggt ctgctgctgc tgaacggcaa gccgttgctg attcgaggcg 2220
ttaaccgtca cgagcatcat cctctgcatg gtcaggtcat ggatgagcag acgatggtgc 2280
aggatatcct gctgatgaag cagaacaact ttaacgccgt gcgctgttcg cattatccga 2340
accatccgct gtggtacacg ctgtgcgacc gctacggcct gtatgtggtg gatgaagcca 2400
atattgaaac ccacggcatg gtgccaatga atcgtctgac cgatgatccg cgctggctac 2460
cggcgatgag cgaacgcgta acgcgaatgg tgcagcgcga tcgtaatcac ccgagtgtga 2520
tcatctggtc gctggggaat gaatcaggcc acggcgctaa tcacgacgcg ctgtatcgct 2580
ggatcaaatc tgtcgatcct tcccgcccgg tgcagtatga aggcggcgga gccgacacca 2640
cggccaccga tattatttgc ccgatgtacg cgcgcgtgga tgaagaccag cccttcccgg 2700
ctgtgccgaa atggtccatc aaaaaatggc tttcgctacc tggagagacg cgcccgctga 2760
tcctttgcga atacgcccac gcgatgggta acagtcttgg cggtttcgct aaatactggc 2820
aggcgtttcg tcagtatccc cgtttacagg gcggcttcgt ctgggactgg gtggatcagt 2880
cgctgattaa atatgatgaa aacggcaacc cgtggtcggc ttacggcggt gattttggcg 2940
atacgccgaa cgatcgccag ttctgtatga acggtctggt ctttgccgac cgcacgccgc 3000
atccagcgct gacggaagca aaacaccagc agcagttttt ccagttccgt ttatccgggc 3060
aaaccatcga agtgaccagc gaatacctgt tccgtcatag cgataacgac ctcctgcact 3120
ggatggtggc gctggatggt aagccgctgg caagcggtga agtgcctctg gatgtcgctc 3180
cacaaggtaa acagttgatt gaactgcctg aactaccgca gccggagagc gccgggcaac 3240

tctggctcac agtacgcgta gtgcaaccga acgcgaccgc atggtcagaa gccgggcaca 3300
tcagcgcctg gcagcagtgg cgtctggcgg aaaacctcag tgtgacgctc cccgccgcgt 3360
cccacgccat cccgcatctg accaccagcg aaatggattt ttgcatcgag ctgggtaaga 3420
agcgttggca atttaaccgc cagtcaggct ttctcccaca gatgtggatt ggcgataaaa 3480
aacaactgct gacgccgctg cgcgatcagt tcacccgtgc accgctggat aacgacattg 3540
gcgtaagtga agcgacccgc attgacccta acgcctgggt cgaacgctgg aaggcggcgg 3600
gccattacca ggccgaagca gcgttgttgc agtgcacggc agatacactt gctgatgcgg 3660
tgctgattac gaccgctcac gcgtggcagc atcaggggaa aaccttattt atcatccgga 3720
aaacctaccg gattgatggt agtggtcaaa tggcgattac cgttgatgtt gaagtggcga 3780
gcgatacacc gcatccggcg cggattggcc tgaactgcca gctggcgcag gtagcagagc 3840
gggtaaactg gctcggatta gggccgcaag aaaactatcc cgaccgcctt actgccgcct 3900
gttttgaccg ctgggatctg ccattgtcag acatgtatac cccgtacgtc ttcccgagcg 3960
aaaacggtct gcgctgcggg acgcgcgaat tgaattatgg cccacaccag tggcgcggcg 4020
acttccagtt caacatcagc cgctacagtc aacagcaact gatggaaacc agccatcgcc 4080
atctgctgca cgcggaagaa ggcacatggc tgaatatcga cggtttccat atggggattg 4140
gtggcgacga ctcctggagc ccgtcagtat cggcggaatt ccagctgagc gccggtcgct 4200
accattacca gttggtctgg tgtcagggga tcccccgggc tgcagccaat atgggatcgg 4260
ccattgaaca agatggattg cacgcaggtt ctccggccgc ttgggtggag aggctattcg 4320
gctatgactg ggcacaacag acaatcggct gctctgatgc cgccgtgttc cggctgtcag 4380
cgcaggggcg cccggttctt tttgtcaaga ccgacctgtc cggtgccctg aatgaactgc 4440
aggacgaggc agcgcggcta tcgtggctgg ccacgacggg cgttccttgc gcagctgtgc 4500
tcgacgttgt cactgaagcg ggaagggact ggctgctatt gggcgaagtg ccggggcagg 4560
atctcctgtc atctcacctt gctcctgccg agaaagtatc catcatggct gatgcaatgc 4620
ggcggctgca tacgcttgat ccggctacct gcccattcga ccaccaagcg aaacatcgca 4680
tcgagcgagc acgtactcgg atggaagccg gtcttgtcga tcaggatgat ctggacgaag 4740
agcatcaggg gctcgcgcca gccgaactgt tcgccaggct caaggcgcgc atgcccgacg 4800
gcgaggatct cgtcgtgacc catggcgatg cctgcttgcc gaatatcatg gtggaaaatg 4860
gccgcttttc tggattcatc gactgtggcc ggctgggtgt ggcggaccgc tatcaggaca 4920
tagcgttggc tacccgtgat attgctgaag agcttggcgg cgaatgggct gaccgcttcc 4980
tcgtgcttta cggtatcgcc gctcccgatt cgcagcgcat cgccttctat cgccttcttg 5040
acgagttctt ctga 5054

## Description

### Technical Field

The present invention relates to a preparation method for cells whose function of expressing wild type genes is deficient or suppressed, antisense promoter insertion vector applicable to preparation of the cells whose function of expressing wild type genes is deficient or suppressed, a gene-trap method using the vector, and the like.

### Prior Arts

Living organisms control the cell proliferation and differentiation via various kinds of signaling molecules. Many of the signaling molecules, such as peptide or steroid etc. and their receptors have been separated and identified so far. Their functional analyses at individual levels have been conducted by producing and using knockout mice. To elucidate expression of what gene populations do these biological stimuli control and how the stimuli alter the cell condition is a biological importance. Large-scale mutant screenings have conventionally been performed for Drosophila, nematode and Zebrafish, using mutagenetic chemicals such as ENU. Subsequently, with an individual phenotype as an index, a mutant involved in a specific biological event is separated and its causal gene is identified, and thus many biological events have been elucidated at molecular levels.

On the other hand, large-scale mutant screenings using individual mice have scarcely been possible because of, for instance, insufficient capacity of experimental animal facilities. Such large-scale screenings for mammals, however, have become possible since the establishment of a gene-trap method (or promoter-trap method) based on insertion mutation of a foreign vector to Embryonic Stem cells (ES cells) (Science 244, 463-465, 1989; Genes & Dev. 5, 1513-1523, 1991; Genes & Dev. 6, 903-918, 1992). Not only a mutated gene can be identified but the individual phenotype can also be analyzed as an advantage of using, especially, ES cells.

Methods such as differential display method and cDNA subtraction method, where vectors are inserted at random, enable to identify genes without depending on expression level of the target gene. But since insertion mutation occurs in an arbitrary manner, a method to select mutations likely to be essential from among various random mutations is needed. Therefore at the time when gene-trap method was first established, a method was applied which embryo-manipulates a trap-clone to produce chimera and then screens its expression pattern. But this method requires lots of embryo manipulation, and what is more, the expression pattern alone could not provide enough information as to whether a gene is the gene of the interest for a resercher.

A vector comprising a fusion gene (β-geo) of β-galactosidase (β-gal) and neomycin resistance gene (neo^{R}) is often used as a selection marker in gene-trap method, but the β-geo vector had a demerit in that by using the vector, only trap-clone of a gene already expressed in ES cells were obtainable. In order to solve the problems, polyA trap method was developed wherein a splice donor sequence is arranged in downstream of neomycin resistance gene linked to phosphoglycerate kinase or to the equivalent strong promoter (Nature 392, 608-611, 1998, Dev. Dyn. 212, 326-333, 1998). This method enables to capture genes that have not been expressed at all before given a stimulus.

The most essential feature of a vector used in the above induction-selection gene-trap is that it has a splice acceptor (SA) sequence to which some kind of reporter gene such as lacZ or GFP is linked, in direct downstream of SA. When this vector is inserted in normal direction into either intron or exon of gene "X", mRNA transcribed from this mutant locus links to SA of the vector from an exon in upstream of the gene "X" insertion site, and becomes "X"-reporter fusion mRNA. With this fusion gene product, the endogenous promoter activity can be monitored. For instance, assuming that "X" is induced or controlled by a certain liquid factor, it results in enhancement or attenuation of X-gal staining or GFP brightness. Hence by selecting and analyzing clones reacting to such liquid factor, a downstream gene for the certain liquid factor can be identified. Moreover, mutant mice obtained through embryo manipulation enable to observe expression pattern of gene "X", as well as to analyze knockout mouse phenotype since insertionally mutated locus is disrupted.

On the other hand, Cre-lox recombinant system for bacteriophage P1 (J. Mol. Biol. (1981) 150, 467-486) and FLP-FRT recombinant system for Yeast Saccharomyces cerevisiae (J. Mol. Biol. (1998) 284, 363-384) are known as effective tools for gene manipulation. Cre recombinase (recombinase enzyme), for example, induces site-specific inter-recombination in between two loxP sequences. When parallel two loxP sequences exist on the same DNA molecule, DNA sequence sandwiched in between the sequences is excised to become a circular molecule (excision reaction). Contrarily, when two loxP sequences exist on different DNA molecules with one of the molecules being circular DNA, the circular DNA is inserted onto another DNA molecule via loxP sequence (insertion reaction). Taking advantage of the insertion reaction, a gene of the interest can be inserted into chromosomes of an animal/plant cell where loxP already exists. But since equivalence of excision-insertion reaction of Cre recombinase deviates to excision reaction side, many of inserted DNAs are re- excised which results in low efficiency.

The above-mentioned wild type loxP sequence consists of 34-base DNA sequence comprising two inverted repeat sequences of 13 bases each and a spacer region sequence of 8 bases which is sandwiched in between said inverted repeat sequences. Recombination of DNA chain is known to take place in the above-mentioned spacer region. Meanwhile, methods are proposed wherein loxP sequence (mutant type) is used, which has a different base sequence from that of the original loxP sequence (wild type) shown as Seq. ID No.1 on the sequence listing, so as to the efficiency of the above-mentioned insertion reaction by recombinase is improved. These methods include such in which lox 71 having mutant loxP sequence shown as Seq. ID.No.2 along with lox 66 having mutant loxP sequence shown as Seq. ID. No.3 are used (Nucleic Acids Research, 1997, Vol.25, No.4, 868-872), and a method utilizing mutant loxP sequence wherein part of a base sequence in spacer region is substituted with some other bases (Japanese Laid-Open Patent Application No.11-196880).

It has previously been reported (Cell, Vol.85, 319-329, May 3, 1996) that a tumor-sensitive gene is isolated by controlling knockout-function of the allelic locus in mammalian cells by using: a gene search vector pLLGSY having SV40-derived antisense promoter that is linked to upstream of a splice acceptor sequence, in inverse direction to the endogenous gene; a transactivator vector pLLTX having in between loxP sequences (wild type) LAP348 sequence capable of activating transcription of the above antisense promoter,; and an expression vector pRSV-cre for Cre recombinase gene which is for excising DNA in between loxP sequences (wild type) in said transactivator vector pLLTX.

Since a gene-trap vector disrupts only one side of the allele by mutation of the insertion gene, homozygotes should be used to observe phenotypes at the time of gene disruption, which homozygotes are obtained by intercrossing chimera mice that are produced from trap-clone of ES cells. In addition, in vitro cell-differentiation systems other than ES cells have great difficulty in disrupting both alleles, therefore use of an antisense promoter has also been considered. Nevertheless, methods capable of efficiently inserting an antisense promoter in the given position of a specific locus remained unknown. The subject of the present invention is to provide the followings that are applicable in in vitro cell-differentiation systems other than ES cells; cells whose function to express normal genes (wild type genes) is deficient or suppressed in the alleles corresponding to trapped genes; the preparation method for the same; gene-trap method utilizing said cells; and antisense promoter insertion vector used in preparation of the cells.

### Summary of the Invention

The present inventors have produced a gene-trap vector and antisense promoter insertion vector. The gene-trap vector is inserted with above-mentioned mutant loxP sequence in upstream of the splice acceptor sequence of a plasmid vector having conventional splice acceptor sequence along with β-geo sequence. Antisense promoter insertion vector can insert a strong promoter in the above mutant loxP sequence site, in inverse direction to the endogenous gene. The present inventors have found that above-mentioned trap vector not only enables to efficiently obtain trap-clones but also enables to forcibly transcript antisense RNA of trapped genes and to disrupt transcripts from wild type genes, by inserting an antisense promoter in a certain position of the mutant locus where a gene-trap vector of trap-clone is inserted in. Here the present invention has accomplished.

The present invention thus relates to: a preparation method for cells whose function of expressing wild type genes is deficient or suppressed, wherein a gene-trap vector is introduced into the cell, trap-clones that are insertionally gene-mutated are selected, and a promoter is inserted in inverse direction to the endogenous gene into a certain position of mutant locus where a gene trap vector of such trap-clone is inserted in, by using antisense promoter insertion vector (Claim 1); a preparation method for cells whose function of expressing wild type genes is deficient or suppressed according to Claim 1, wherein the gene-trap vector has a splice acceptor sequence or a splice donor sequence (Claim 2); a preparation method for cells whose function of expressing wild type genes is deficient or suppressed according to Claim 2, wherein the gene-trap vector has a selection marker gene or a reporter gene that are linked to downstream of the splice acceptor sequence or upstream of the splice donor sequence (Claim 3); a preparation method for cells whose function of expressing wild type genes is deficient or suppressed according to Claim 3, wherein the gene-trap vector has, via IRES, a selection marker gene or a reporter gene that are linked to downstream of the splice acceptor sequence or upstream of the splice donor sequence (Claim 4); a preparation method for cells whose function of expressing wild type genes is deficient or suppressed according to either of Claims 3 or 4, wherein the selection marker gene is one or more genes selected from among neomycin resistance gene, puromycin resistance gene, hygromycin resistance gene, or diphtheriatoxin resistance gene (Claim 5); a preparation method for cells whose function of expressing wild type genes is deficient or suppressed according to any one of Claims 1 to 5, wherein the gene-trap vector has a mutant inverted repeat sequence which is a mutant of wild type inverted repeat sequence recognized by recombinase (Claim 6); a preparation method for cells whose function of expressing wild type genes is deficient or suppressed according to Claim 6, wherein a single mutant inverted repeat sequence is in upstream region of the splice acceptor sequence (Claim 7); a preparation method for cells whose function of expressing wild type genes is deficient or suppressed according to Claim 6, wherein two mutant inverted repeat sequences are both in upstream region of the splice acceptor sequence (Claim 8); a preparation method for cells whose function of expressing wild type genes is deficient or suppressed according to Claim 6, wherein mutant inverted repeat sequences are respectively in upstream region of the splice acceptor sequence and in downstream region of the selection marker gene (Claim 9); a preparation method for cells whose function of expressing wild type genes is deficient or suppressed according to either of Claims 8 or 9, wherein mutant inverted repeat sequences are identical with or different from each other (Claim 10); a preparation method for cells whose function of expressing wild type genes is deficient or suppressed according to Claim 6, wherein a mutant inverted repeat sequence and a wild type inverted repeat sequence are both in upstream region of the splice acceptor sequence in an arbitrary manner (Claim 11); a preparation method for cells whose function of expressing wild type genes is deficient or suppressed according to Claim 6, wherein either of a mutant inverted repeat sequence or a wild type inverted repeat sequence is in upstream region of the splice acceptor sequence and another in down stream region of the selection marker gene (Claim 12); a preparation method for cells whose function of expressing wild type genes is deficient or suppressed according to any one of Claims 1 to 12, wherein antisense promoter insertion vector has a mutant inverted repeat sequence which is a mutant of wild type inverted repeat sequence recognized by recombinase, into which sequence a promoter can be inserted in inverse direction to the endogenous gene at a certain position of the mutant locus where a gene-trap vector is inserted in (Claim 13); a preparation method for cells whose function of expressing wild type genes is deficient or suppressed according to Claim 13, wherein different mutant inverted repeat sequences are respectively in upstream and downstream regions of the promoter which is inserted in inverse direction to the endogenous gene (Claim 14); a preparation method for cells whose function of expressing wild type genes is deficient or suppressed according to Claim 13, wherein identical mutant inverted repeat sequences are respectively in upstream and downstream regions of the promoter which is inserted in inverse direction to the endogenous gene (Claim 15); a preparation method for cells whose function of expressing wild type genes is deficient or suppressed according to Claim 13, wherein either of a mutant inverted repeat sequence or a wild inverted repeat sequence is in upstream region of the promoter which is inserted in inverse direction to the endogenous gene and another is in downstream of said promoter (Claim 16); a preparation method for cells whose function of expressing wild type genes is deficient or suppressed according to any one of Claims 6 to 16, wherein a mutant inverted repeat sequence is a mutant of E.Coli P1 pharge-derived wild type loxP sequence or Yeast Saccharomyces cerevisiae-derived wild type FRT sequence (Claim 17); a preparation method for cells whose function of expressing wild type genes is deficient or suppressed according to any one of Claims 6 to 17, wherein a mutant inverted repeat sequence has a mutation in an inverted repeat region (Claim 18); a preparation method for cells whose function of expressing wild type genes is deficient or suppressed according to Claim 18, wherein the mutant inverted repeat sequence whose mutation is in the inverted repeat region is lox71 sequence which has a base sequence shown as Seq.ID No.2 and/or lox66 sequence which has a base sequence shown as Seq.ID No.3 (Claim 19); a preparation method for cells whose function of expressing wild type genes is deficient or suppressed according to any one of Claims 6 to 17, wherein the mutant inverted repeat sequence has a mutation in the spacer region which is in between the inverted repeat regions (Claim 20); a preparation method for cells whose function of expressing wild type genes is deficient or suppressed according to any one of Claims 1 to 20, wherein a different selection marker gene from that of the gene-trap vector is linked to downstream of the promoter which is inserted in inverse direction to the endogenous gene (Claim 21); a preparation method for cells whose function of expressing wild type genes is deficient or suppressed according to Claim 21, wherein the selection marker gene linked to downstream of the promoter which is inserted in inverse direction to the endogenous gene is puromycin resistance gene (Claim 22); a preparation method for cells whose function of expressing wild type genes is deficient or suppressed according to any one of Claims 13 to 22, wherein antisense promoter insertion vector has a selection marker gene of the gene-trap vector, and a negative selection marker gene different from the selection marker gene linked to downstream of the promoter inserted in inverse direction to the endogenous gene, in upstream of said promoter inserted in inverse direction to the endogenous gene, and in same or inverse direction to said promoter, via a mutant or a wild type inverted repeat sequence (Claim 23); a preparation method for cells whose function of expressing wild type genes is deficient or suppressed according to Claim 23, wherein the negative selection marker gene is thymidine kinase gene or diphtheriatoxin resistance gene that are linked to the promoter for phosphoglycerate kinase (Claim 24); a preparation method for cells whose function of expressing wild type genes is deficient or suppressed according to any one of Claims 1 to 24, wherein the promoter inserted in inverse direction to the endogenous gene is a promoter for phosphoglycerate kinase (Claim 25); a preparation method for cells whose function of expressing wild type genes is deficient or suppressed according to any one of Claims 2 to 25, wherein a certain position in the mutant locus is upstream region of the splice acceptor sequence (Claim 26); a preparation method for cells whose function of expressing wild type genes is deficient or suppressed according to any one of Claims 1 to 26, characterized in inserting a promoter in inverse direction to the endogenous gene by expressing recombinase in the cells (Claim 27); and a preparation method for cells whose function of expressing wild type genes is deficient or suppressed according to any one of Claims 1 to 27, wherein the cells are ES cells (Claim 28).

The present invention further relates to cells capable of transcribing antisense RNA to the trapped genes, obtained by the preparation method for cells whose function of expressing wild type genes is deficient or suppressed according to any one of Claims 1 to 28 (Claim 29).

The present invention still further relates to antisense promoter insertion vector characterized in having a promoter capable of being inserted in inverse direction to the endogenous gene at a certain position of a mutant locus where the gene-trap vector of insertionally gene mutated trap-clone is inserted in (Claim 30); antisense promoter insertion vector according to Claim 30, characterized in having a mutant inverted repeat sequence which is a mutant of a wild type inverted repeat sequence recognized by recombinase (Claim 31); antisense promoter insertion vector according to Claim 31, characterized in having different mutant inverted repeat sequences respectively in upstream and downstream regions of the promoter which is inserted in inverse direction to the endogenous gene (Claim 32); antisense promoter insertion vector according to Claim 31, characterized in having identical mutant inverted repeat sequences respectively in upstream and downstream regions of the promoter which is inserted in inverse direction to the endogenous gene (Claim 33); antisense promoter insertion vector according to Claim 31, characterized in having a single mutant inverted repeat sequence and a single wild type inverted repeat sequence, wherein either of the sequences is in upstream region of the promoter which is inserted in inverse direction to the endogenous gene and another in downstream region of said promoter (Claim 34); antisense promoter insertion vector according to any one of Claims 31 to 34, wherein a mutant inverted repeat sequence is a mutant of E.Coli P1 pharge-derived wild type loxP sequence or Yeast Saccharomyces cerevisiae-derived wild type FRT sequence (Claim 35); antisense promoter insertion vector according to any one of Claims 31 to 35, wherein a mutant inverted repeat sequence has a mutation in an inverted repeat region (Claim 36); antisense promoter insertion vector according to Claim 36, wherein a mutant inverted repeat sequence, whose mutation is in the inverted repeat region, is lox71 sequence which has a base sequence shown as Seq.ID No.2 and/or lox66 sequence which has a base sequence shown as Seq.ID No.3 (Claim 37); antisense promoter insertion vector according to any one of Claims 31 to 35, wherein the mutant inverted repeat sequence has a mutation in the spacer region which is in between the inverted repeat regions (Claim 38); antisense promoter insertion vector according to any one of Claims 30 to 38, wherein a different selection marker gene from that of the gene-trap vector is linked to downstream of the promoter which is inserted in inverse direction to the endogenous gene (Claim 39); antisense promoter insertion vector according to Claim 39, wherein the selection marker gene linked to downstream of the promoter which is inserted in inverse direction to the endogenous gene is puromycin resistance gene (Claim 40); antisense promoter insertion vector according to any one of Claims 30 to 40, characterized in having, in upstream region of the promoter which is inserted in inverse direction to the endogenous gene, said promoter and a negative selection marker gene in same or inverse direction to said promoter via a mutant or a wild type inverted repeat sequence (Claim 41); antisense promoter insertion vector according to Claim 41, wherein the negative selection marker gene is thymidine kinase gene or diphtheriatoxin resistance gene that are linked to the promoter for phosphoglycerate kinase (Claim 42); and antisense promoter insertion vector according to any one of Claims 30 to 42, wherein the promoter inserted in inverse direction to the endogenous gene is a promoter for phosphoglycerate kinase (Claim 43).

The invention also relates to a gene trap method characterized in using antisense promoter insertion vector according to any one of Claims 30 to 43 (Claim 44).

The invention also relates to a cell treatment method to delete or suppress expression of a specific gene in the cells, characterized in using antisense promoter insertion vector according to any one of Claims 30 to 43 (Claim 45)

The invention also relates to a knockout-like mouse whose gene expression is deficient or suppressed, characterized in being obtained by introducing antisense promoter insertion vector according to any one of Claims 30 to 43 into ES cells, and by microinjecting mice blastocysts with the ES cells (Claim 46).

### Brief Description of the Drawings

Fig.1 is a schematic drawing showing insertion of a promoter into a certain position of the mutant locus where a gene trap vector of trap-clone is inserted in, by using antisense promoter insertion vector of the present invention. The promoter is inserted in inverse direction to the endogenous gene.
Fig.2 is a schematic drawing showing insertion of a promoter of different mode.
Fig.3 is a schematic drawing showing insertion of a promoter of different mode.
Fig.4 is a schematic drawing showing insertion of a promoter of different mode.
Fig.5 is a schematic drawing showing insertion of a promoter of different mode.
Fig.6 is a schematic drawing showing insertion of a promoter of different mode.
Fig.7 is a schematic drawing showing insertion of a promoter of different mode.
Fig.8 is a schematic drawing showing insertion of a promoter of different mode.
Fig.9 is a drawing showing a set consisting the antisense gene-trap vector of the present invention and a conventional gene-trap vector.

### Best Mode for Carrying out the Invention

The preparation method of the present invention for cells whose function to express wild type genes are deficient or suppressed is not specifically limited as long as a method includes and enables: introducing gene-trap vector into the cell; selecting trap-clone which is insertionally gene-mutated; inserting a promoter in inverse direction to the endogenous gene into a certain position of the mutant locus where a gene-trap vector of the trap-clone is inserted in, by using antisense promoter insertion vector. Here, cells whose function to express wild type genes are deficient or suppressed mean cells whose function to express normal genes in the alleles corresponding to trapped genes are deficient or suppressed. The deficiency or suppression of the expression function is brought about by hybridization of the transcripts from antisense promoter (antisense RNA) and the transcripts from wild-type genes (sense RNA). The cells used are not specifically limited and animal or plant cells are the examples. ES cell is a good example of animal cells, while the present invention is characterized in being applicable in other cell-differentiation systems in vitro than ES cells.

Any gene-trap vector can be used for the above-mentioned gene-trap vector as long as it can cause insertional gene-mutation when introduced into the cells. Still a gene-trap vector having splice acceptor sequence is preferable. More preferable is a gene-trap vector to which a selection marker gene or a reporter gene is linked to downstream of the splice acceptor sequence, particularly a gene-trap vector to which a selection marker gene is linked via IRES (internal ribosomal entry site). In addition, a vector having splicing donor sequence in downstream of a selection marker gene linked to a strong promoter is also preferable. As the splice acceptor sequence, any DNA sequence will suffice as long as it can form splice acceptor site on its 5' terminal and one specific example is the splice acceptor sequence of Engrailed-2. By having splice acceptor sequence, 3'-terminal exon of vector-insertion site is spliced into a selection marker gene, and as a result a selection marker gene or a reporter gene can be transcribed as a fusion gene with the endogenous gene fragment. Any DNA sequence will suffice as the above splice donor sequence as long as it can donate splicing on its 3'-terminal, and splice donor sequence of mouse hprt (hypoxanthine phosphoribosyl transferase) gene is among the specific examples. By having splice donor sequence, splicing into endogenous exon on 3' terminal of vector insertion site takes place, and the selection marker gene can be transcribed as a fusion gene with the endogenous gene fragment. Further, any selection marker gene may be used as long as it can screen gene-trap-clones. The specific examples include neomycin resistance gene, puromycin resistance gene, hygromycin resistance gene, diphtheriatoxin resistance gene, fusion gene of β-gal and neo^{R} (β-geo). When such selection marker gene is linked via IRES, the translation starts with AUG in downstream of IRES as an initiation codon.

Further, as a gene-trap vector of the present invention, that having a mutant inverted repeat sequence which is a mutant of wild type inverted repeat sequence recognized by recombinase is preferable. This is because such gene-trap vector can efficiently insert a promoter in inverse direction to the endogenous gene, cooperating with antisense promoter insertion vector. Such gene-trap vectors having the above mutant inverted repeat sequences include: a vector having a single mutant inverted repeat sequence in upstream region of splice acceptor sequence; a vector having two identical or different mutant inverted repeat sequences, both in upstream region of splice acceptor sequence; a vector having two identical or different mutant inverted repeat sequences, one in upstream region of splice acceptor sequence and another in downstream region of selection marker gene respectively; a vector having a single mutant inverted repeat sequence and a single wild type inverted repeat sequence, both in upstream region of splice acceptor sequence in arbitrary order; and a vector having a single mutant inverted repeat sequence and a single wild type inverted repeat sequence, with one of the sequences in upstream region of splice acceptor sequence, and another in downstream region of selection marker gene.

Examples of above-mentioned wild type inverted repeat sequence recognized by recombinase are loxP sequence recognized by Cre recombinase of bacteriophage P1 and FRT sequence recognized by FLP recombinase of Yeast Saccharomyces cerevisiae. And a mutant inverted repeat sequence which is a mutant of wild type inverted repeat sequence means a mutant sequence of the wild type inverted repeat sequence whose one or more bases are substituted, deleted, added or etc, and that such mutation alters the substrate specificity in a specific DNA recombinant reaction by recombinase. The followings are exemplified as specific examples of the mutant inverted repeat sequences: lox 71 having mutant loxP sequence shown as Seq. ID. No.2 in which a part of the base sequence, in the inverted repeat sequence region of wild type loxP sequence shown as Seq.ID.No.1, is substituted with some other bases; lox 66 having mutant loxP sequence shown as Seq. ID. No.3 (Nucleic Acids Research, 1997, Vol.25, No.4, 868-872); a mutant loxP sequence where a part of base sequence in the spacer region of the above wild type loxP sequence is substituted with some other bases (Japanese Laid-Open Patent Application No.11-196880); a mutant FRT sequence shown as Seq. ID. No.5, where a part of base sequence in the inverted repeat sequence region of wild type FRT sequence shown as Seq. ID. No.4 is substituted with some other bases (J. Mol. Biol .(1998) 284, 363-384); and the like.

Any vector will suffice as antisense promoter insertion vector of the present invention as long as it carries a promoter that can be inserted in inverse direction to the endogenous gene at a certain position of the mutant locus where above gene-trap vector of the insertionally gene mutated trap-clone is inserted in. Among them, a vector is preferable that has mutant inverted repeat sequence which is a mutant of wild type inverted repeat sequence recognized by recombinase. The examples of vectors carrying antisense promoters that have such mutant inverted repeat sequence are: a vector having different or identical mutant inverted repeat sequences respectively in upstream and in downstream regions of a promoter which is to be inserted in inverse direction to the endogenous gene; and a vector having a single mutant inverted repeat sequence and a single wild type inverted repeat sequence, with one of the sequences in upstream of a promoter which is inserted in inverse direction to the endogenous gene and another in downstream of said promoter.

Antisense promoter insertion vector preferable for the present invention is the one to which a different selection marker gene from that of a gene-trap vector is linked to downstream of a promoter which is inserted in inverse direction to the endogenous gene. This is because it can be confirmed that an antisense promoter is introduced into a certain position of the mutant locus where a gene-trap vector of trap-clone is inserted in. Such selection marker gene shall not be limited in particular, but such as puromycin resistance gene can be exemplified. In addition, it is preferable to place a negative selection marker gene in antisense promoter insertion vector in upstream region of a promoter which is inserted in inverse direction to the endogenous gene, via a mutant or a wild type inverted repeat sequence. Here, the negative selection marker gene is different from a selection marker gene of the gene-trap vector or from a selection marker gene linked to downstream of above-mentioned promoter, and is placed in same or in inverse direction to said promoter. This enables more precise confirmation as to whether the antisense promoter is introduced into a certain position of the mutant locus where a gene-trap vector of trap-clone is inserted in. Such negative selection marker gene will not specifically be limited, and by using, for instance, thymidine kinase gene, diphtheriatoxin resistance gene, etc. linked to a promoter of phosphoglycerate kinase, the inserted clones can be removed with ganciclovir without being affected by recombinase.

Further, as a promoter which is to be inserted in inverse direction to the endogenous gene and which is for antisense promoter insertion vector of the present invention, any promoter will suffice as long as it can transcript an antisense RNA of trap-gene on 5' side of the certain position of a mutant locus where a gene-trap vector of trap-clone is inserted in. Among them, a promoter with strong activity is preferable. Examples of the strong promoters include: a promoter for mouse phosphoglycerate kinase; RNA polymerase II promoter; an early promoter for SV40; a late promoter for SV40; a major late promoter for adenovirus; metallothionein promoter; LTR for retrovirus; thymidine kinase promoter for herpes simplex virus; and the like. Further, trap-gene expression can be controlled by using a promoter with switch function such as a promoter for large T antigen gene of the above-mentioned SV40 thermo-sensitive mutant strain tsA 58. In addition, an enhancer can be placed in upstream of a promoter.

As a certain position of mutant locus of the present invention, where a gene-trap vector of trap clone is inserted in, any position will do as long as a promoter, which is inserted in inverse direction to the endogenous gene by using antisense promoter insertion vector, can transcript antisense RNA of a trap gene. Still, upstream region of splice acceptor sequence is preferable. In addition, any method, for inserting said promoter to the given position of mutant locus where the gene-trap vector is inserted, can be applied as long as it is a conventionally known DNA insertion technique. Recombinase is preferred to be used for insertion of said promoter when gene-trap vector has a mutant inverted repeat sequence. Such recombinase will be allowed to exist in the cells by being expressed by a recombinase gene expressing-vector etc.

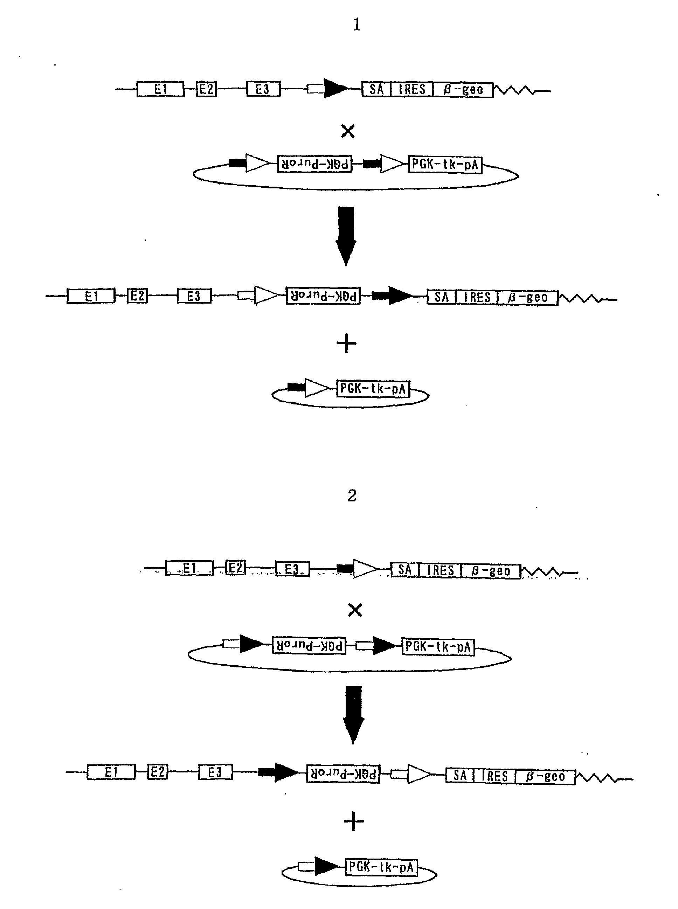

Steps in the preparation method of the present invention for cells whose function of expressing wild type genes is deficient or suppressed will be explained as follows: Aforementioned gene-trap vector is introduced into a cell; Trap-clone, which is insertionally gene mutated by integrating said vector into chromosomal DNA of the cell, is selected by selection marker gene expression; antisense promoter insertion vector and Cre recombinase expression vector are introduced into the trap-clone; Then the promoter is inserted, in inverse direction to the endogenous gene, into a certain position of the mutant locus where the gene-trap vector is inserted in. A conventionally known method may be used in introducing circular DNA molecules into animal cells. Introduction methods for the above vectors into the cells are not limited in particular and include, for example, electroporation, calcium-phosphate coprecipitation, DEAE-dextran, Lipofection, Helios Gene Gun and the like. The above-mentioned antisense promoter-introduction will be explained with examples referring to Figs.1 to 8. Figs.1 to 8 are schematic drawings showing: a mutant locus where a gene-trap vector is inserted in downstream of exon 3 (E3), to which gene-trap vector a selection marker gene β-geo is linked in downstream of the splice acceptor sequence (SA) via IRES; and the introduction of antisense promoter using phosphoglycerate kinase promoter (PGK-PuroR) to which puromycin resistance gene is linked in downstream of the mutant locus, and using antisense promoter-introduced vector having thymidine kinase gene and poly A (PGK-tk-pA) linked to downstream of phosphoglycerate kinase promoter.

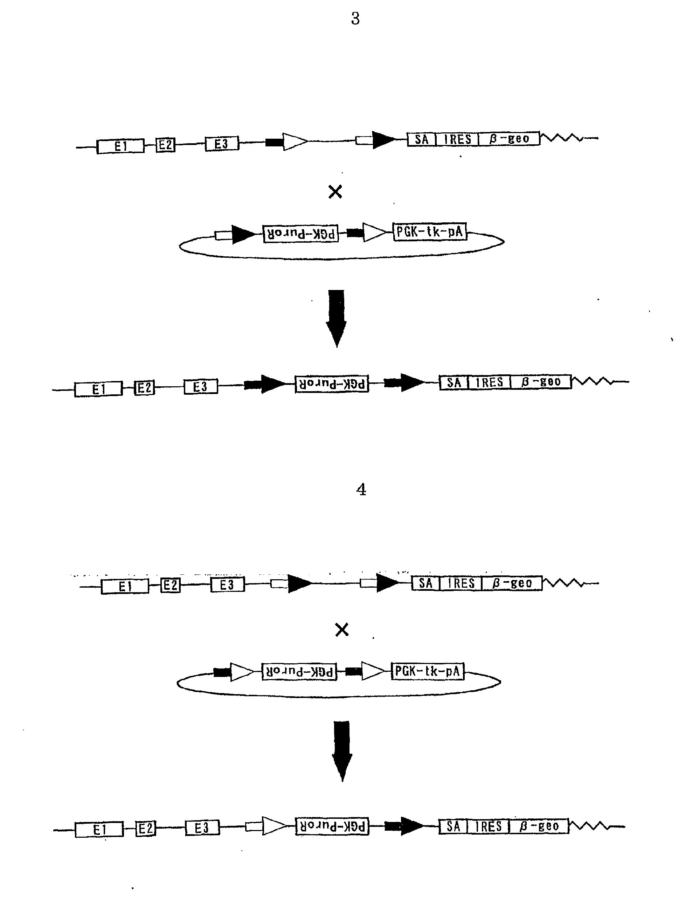

Fig.1 shows the case using a gene-trap vector having a single mutant inverted repeat sequence (lox71) in upstream region of the splice acceptor sequence and antisense promoter insertion vector having identical mutant inverted repeat sequences (lox66), in upstream and downstream regions of the antisense promoter respectively. Fig.2 shows the case using a gene-trap vector having a single mutant inverted repeat sequence (lox66) in upstream region of the splice acceptor sequence, and antisense promoter insertion vector having identical mutant inverted repeat sequences (lox71) in upstream and downstream regions of the antisense promoter respectively.

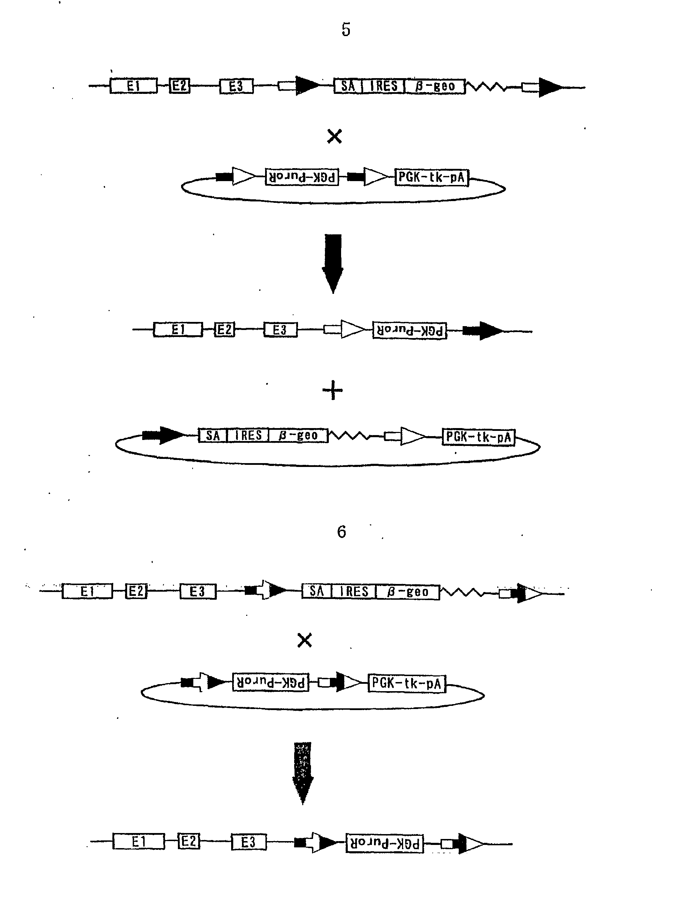

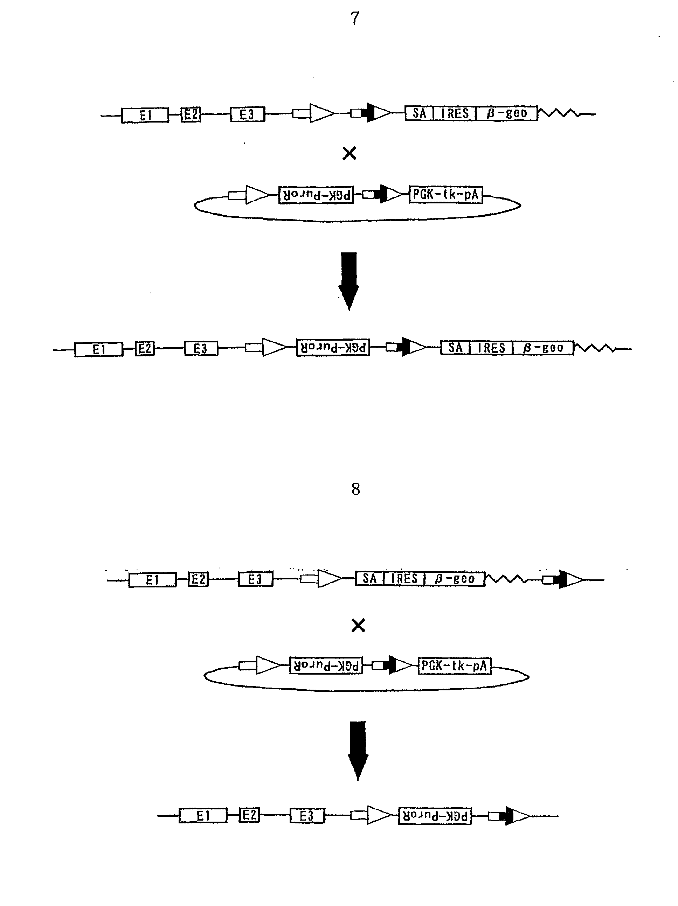

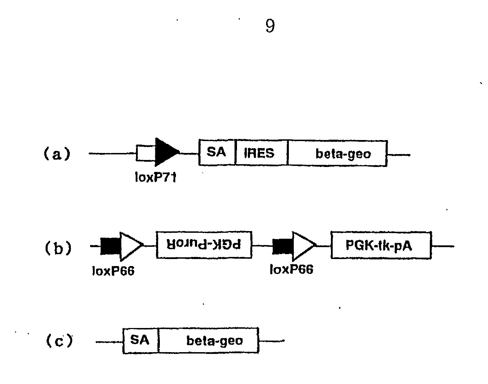

Fig. 3 shows the case using a gene-trap vector having different mutant inverted repeat sequences (lox66 and lox71) in upstream region of the splice acceptor sequence, and antisense promoter insertion vector having different mutant inverted repeat sequences (lox71 and lox66) in upstream and downstream regions of the antisense promoter respectively. Fig.4 shows the case using a gene-trap vector having two mutant inverted repeat sequences (lox71) in upstream region of the splice acceptor sequence, and antisense promoter insertion vector having identical mutant inverted repeat sequences (lox66) in upstream and downstream regions of the antisense promoter respectively. Fig.5 shows the case using a gene-trap vector having mutant inverted repeat sequences (lox71) in upstream region of the splice acceptor sequence and in downstream region of the selection marker gene respectively, along with antisense promoter insertion vector having identical mutant inverted repeat sequences (lox66) in upstream and downstream regions of the antisense promoter respectively.

Fig.6 shows the case using a gene-trap vector having different mutant inverted repeat sequences (loxP whose spacer region is mutated) in upstream region of the splice acceptor sequence and in downstream region of the selection marker gene respectively, along with antisense promoter insertion vector having different mutant inverted repeat sequences (loxP whose spacer region is mutated) in upstream and downstream regions of the antisense promoter respectively. Fig.7 shows the case using a gene-trap vector having wild type inverted repeat sequence (loxP) and mutant inverted repeat sequence (loxP whose spacer region is mutated) both in upstream region of the splice acceptor sequence along with antisense promoter insertion vector having wild type inverted repeat sequence (loxP) in upstream region and mutant inverted repeat sequence (loxP whose spacer region is mutated) in downstream region of the antisense promoter, respectively. And Fig.8 shows the case using a gene-trap vector having wild type inverted repeat sequence (loxP) and mutant inverted repeat sequence (loxP whose spacer region is mutated) in upstream region of the splice acceptor sequence and in downstream region of the selection marker gene, respectively, along with antisense promoter insertion vector having wild type inverted repeat sequence (loxP) in upstream region and mutant inverted repeat sequence (loxP whose spacer region is mutated) in downstream of the antisense promoter respectively.

Use of antisense promoter insertion vector of the present invention, in particular, use of the antisense gene-trap vector consisting of above described gene-trap vector and antisense promoter insertion vector of the present invention, enables to delete or suppress expression of a specific gene within the cells. As a result of this, a functional screening can be performed, thus enabling identification of genes inevitable for differentiation etc. without using homozygotes such as conventional knockout mice and the like. Further, knockout-like mice can be generated, whose trap-gene expression is deficient or suppressed, through the process of introducing the above antisense gene-trap vector into ES cells and microinjecting mouse blastocysts with the ES cells. Taken together, cell lines including ES cells or mice-individuals prepared by the method of the present invention can be served as biological materials for human disease model. In addition, when the cell preparation method of the present invention is applied to human cells, expression of a specific gene can be suppressed, and at the same time other kind of genes can be expressed. Hence, application of somatic cells to gene therapy can be expected.

The present invention will now be described in more detail with the examples below, but the technical scope of the invention shall not be limited to these examples.

### Example 1 (producing a gene-trap vector)

About 0.4kb BamHI-Not I fragment (including a part of 1^{st} intron and a part of 2^{nd} exon) of mouse Engrailed 2 gene (En 2) was inserted in between Sma I-Not I sites of plasmid pBluescript (pBS)-SK⁻ including synthesized lox71 sequence (Nucleic Acids Research, 1997, Vol.25, No.4, 868-872). Said plasmid is a gift from Professor Yamamura of Kumamoto University. Xho I-Not I adapter (tcg agc ggc cgc) was added to about 5.5kb Not I-Xho I fragment of plasmid pIRES beta-geo and the total fragment was inserted into Not I site of plasmid plox71 SA including the splice acceptor (SA). About 6kb Sal I fragment of this plasmid plox71 SA IRES beta-geo was inserted in between Sal I-Xho I sites of pBluescript II (Stratagene). BamHI-XhoI adaptor (gat ccc gct cga gcg & ccg ctc gag cgg) was added to about 1.2kb Sal I-BamHI of plasmid pT1 (Sal I) and the total fragment was inserted into Sal I site of the plasmid pSal I/lox71 SA IRES beta-geo, to obtain gene-trap vector pEn/lox71/SA IRES beta-geo/pA. A part of the sequence of this gene-trap vector (Fig.9a) is shown as Seq. No.6.

### Example 2 (Production of antisense promoter insertion vector)

1.7kb Sal I fragment of plasmid pCre-Pac was inserted into Sal I site of pBluescript II (Stratagene). 1.2kb Sal I-Sma I fragment of this plasmid pBSpuro was inserted in between Xba I-Sma I sites of plasmid plox66 which includes synthesized lox66 sequence, a gift from Professor Yamamura of Kumamoto University, to obtain plasmid plox66 PGK-puro. Meanwhile, 2.8kb EcoR I-Sal I fragment of plasmid pGK-tk was inserted in between plox66 Sma I-Xba I sites. Then 3.0kb Not I-Sal I fragment of plasmid plox66 PGK-puro was inserted in between Hind III-Xho I sites of the plasmid plox66 PGK-tk. Antisense promoter insertion vector, plox66 PGK-puro-tk was thus produced (Fig.9B).

### Example 3 (Introduction of gene-trap vector)

In attempt to compare a gene-trap vector produced in Example 1 and a conventional trap-vector (Fig.9c), comparison was made as follows using ES cells with which many experiments have been conducted and thus many results have been obtained. Mouse-derived ES cells cultured in DMEM containing 15% FCS (Fetal Calf Serum) were trypsin-treated for 15-20 min, then centrifuged at 1200rpm for 3 min. The supernatant was removed and the ES cells were suspended on adding 10ml PBS (Phosphate Buffered Saline). The ES cell suspension (1×10⁸) was again centrifuged at 1200rpm for 3 min. The supernatant was removed and pre-linearized gene-trap vector produced according to Example 1 or a conventional trap-vector, 100µg each, was added to the centrifugate. Cold PBS was added to make 0.8ml in final for resuspension, and electroporation at 0.8kV, 3µF was conducted. One tenth of the cells were spread on a 10cm-plate and trap-clones were selected by G418. 252 colonies were obtained when using the gene-trap vector produced according to Example 1, whereas only 80-100 colonies, which is less than half of the gene-trap vector case, were obtained when trap-clones introduced with the conventional trap vector were used.

96 colonies were separated from above 252 colonies. Then X-gal stain (X-gal with the final concentration of 1mg/ml: 5mM potassium ferrocyanide, 5mM potassium ferricyanide, 2mM MgCl₂, 5mM EGTA, 0.02% NP40, 0.1% phosphate buffer; pH7.3) was added to the remaining 156 colonies and were stained at 37°C for 2 days in the dark. As a result, 127 were stained out of 156 colonies and the remaining 29 (18%) were unable to be visually observed. About 30% of colonies were not stained when using the conventional trap vector. That some of the clones were not stained is thought because reporter gene was not expressed due to the generation of fusion protein of the reporter gene product and the trapped gene product. Taken together, the gene-trap vector of the present invention has higher efficiency in obtaining trap clones than the conventional one. What is more, reporter genes can be expressed with high sensitivity.

### Example 4 (Introduction of the antisense promoter)

Cells (7×10⁶) of 10 clones among 96 clones separated according to Example 3, including 3 clones whose trapped gene loci were cloned by 5' RACE, were respectively suspended in PBS. 20µg of the transient expression vector of Cre recombinase (pCre-Pac) and 20µg of antisense promoter insertion vector (plox66 PGK-puro-tk) were added to the suspensions to 0.8ml, followed by electroporation at 0.24kV, 500µF. The cells were plated on two 10cm-glass plates and were replaced onto a selection medium containing 1µg/ml puromysin and 2µM ganciclovir 2 hours later, then they were cultured for 2 days. Subsequently, the cultured cells were replaced onto a selection medium only containing 2µM gancyclovir and were cultured for 5 more days. Average of 70 colonies were obtained as a result from each of 10 clones. Whether vectors carrying antisense promoters were integrated into these colonies obtained was confirmed by Southern blotting. To see whether transcription from wild type alleles was suppressed, Northern blotting was performed for 3 clones to which trapped loci were cloned by 5' RACE and suppression of expression was revealed to be 30-90%.

### Industrial Applicability

The present invention enables to prepare cells that are applicable for in vitro cell-differentiation systems other than ES cells, and whose function for expressing normal genes (wild type genes) in the alleles corresponding to trapped genes is deficient or suppressed. In addition cell lines such as ES cells or mice individuals prepared by the present invention can be served as biological materials for human disease model. Also when the preparation method of cells of the present invention is applied to human cells, expression of a specific gene can be suppressed and at the same time, some other gene can be expressed. Therefore, somatic cells are expected to be applied to gene therapy.

## Claims

1. A preparation method for cells whose function of expressing wild type genes is deficient or suppressed, wherein a gene-trap vector is introduced into the cell, trap-clones that are insertionally gene-mutated are selected, and a promoter is inserted in inverse direction to the endogenous gene into a certain position of mutant locus where a gene trap vector of such trap-clone is inserted in, by using antisense promoter insertion vector.

2. A preparation method for cells whose function of expressing wild type genes is deficient or suppressed according to Claim 1, wherein the gene-trap vector has a splice acceptor sequence or a splice donor sequence.

3. A preparation method for cells whose function of expressing wild type genes is deficient or suppressed according to Claim 2, wherein the gene-trap vector has a selection marker gene or a reporter gene that are linked to downstream of the splice acceptor sequence or upstream of the splice donor sequence.

4. A preparation method for cells whose function of expressing wild type genes is deficient or suppressed according to Claim 3, wherein the gene-trap vector has, via IRES, a selection marker gene or a reporter gene that are linked to downstream of the splice acceptor sequence or upstream of the splice donor sequence.

5. A preparation method for cells whose function of expressing wild type genes is deficient or suppressed according to either of Claims 3 or 4, wherein the selection marker gene is one or more genes selected from among neomycin resistance gene, puromycin resistance gene, hygromycin resistance gene, or diphtheriatoxin resistance gene.

6. A preparation method for cells whose function of expressing wild type genes is deficient or suppressed according to any one of Claims 1 to 5, wherein the gene-trap vector has a mutant inverted repeat sequence which is a mutant of wild type inverted repeat sequence recognized by recombinase.

7. A preparation method for cells whose function of expressing wild type genes is deficient or suppressed according to Claim 6, wherein a single mutant inverted repeat sequence is in upstream region of the splice acceptor sequence.

8. A preparation method for cells whose function of expressing wild type genes is deficient or suppressed according to Claim 6, wherein two mutant inverted repeat sequences are both in upstream region of the splice acceptor sequence.

9. A preparation method for cells whose function of expressing wild type genes is deficient or suppressed according to Claim 6, wherein mutant inverted repeat sequences are respectively in upstream region of the splice acceptor sequence and in downstream region of the selection marker gene.

10. A preparation method for cells whose function of expressing wild type genes is deficient or suppressed according to either of Claims 8 or 9, wherein mutant inverted repeat sequences are identical with or different from each other.

11. A preparation method for cells whose function of expressing wild type genes is deficient or suppressed according to Claim 6, wherein a mutant inverted repeat sequence and a wild type inverted repeat sequence are both in upstream region of the splice acceptor sequence in an arbitrary manner.

12. A preparation method for cells whose function of expressing wild type genes is deficient or suppressed according to Claim 6, wherein either of a mutant inverted repeat sequence or a wild type inverted repeat sequence is in upstream region of the splice acceptor sequence and another in downstream region of the selection marker gene.

13. A preparation method for cells whose function of expressing wild type genes is deficient or suppressed according to any one of Claims 1 to 12, wherein antisense promoter insertion vector has a mutant inverted repeat sequence which is a mutant of wild type inverted repeat sequence recognized by recombinase, and into which sequence a promoter can be inserted in inverse direction to the endogenous gene at a certain position of the mutant locus where a gene-trap vector is inserted in.

14. A preparation method for cells whose function of expressing wild type genes is deficient or suppressed according to Claim 13, wherein different mutant inverted repeat sequences are respectively in upstream and downstream regions of the promoter which is inserted in inverse direction to the endogenous gene.

15. A preparation method for cells whose function of expressing wild type genes is deficient or suppressed according to Claim 13, wherein identical mutant inverted repeat sequences are respectively in upstream and downstream regions of the promoter which is inserted in inverse direction to the endogenous gene.

16. A preparation method for cells whose function of expressing wild type genes is deficient or suppressed according to Claim 13, wherein either of a mutant inverted repeat sequence or a wild inverted repeat sequence is in upstream region of the promoter which is inserted in inverse direction to the endogenous gene and another in downstream of said promoter.

17. A preparation method for cells whose function of expressing wild type genes is deficient or suppressed according to any one of Claims 6 to 16, wherein a mutant inverted repeat sequence is a mutant of E.Coli P1 pharge-derived wild type loxP sequence or Yeast Saccharomyces cerevisiae-derived wild type FRT sequence.

18. A preparation method for cells whose function of expressing wild type genes is deficient or suppressed according to any one of Claims 6 to 17, wherein a mutant inverted repeat sequence has a mutation in an inverted repeat region.

19. A preparation method for cells whose function of expressing wild type genes is deficient or suppressed according to Claim 18, wherein the mutant inverted repeat sequence whose mutation is in the inverted repeat region is lox71 sequence which has a base sequence shown as Seq.ID No.2 and/or lox66 sequence which has a base sequence shown as Seq.ID No.3.

20. A preparation method for cells whose function of expressing wild type genes is deficient or suppressed according to any one of Claims 6 to 17, wherein the mutant inverted repeat sequence has a mutation in the spacer region which is in between the inverted repeat regions.

21. A preparation method for cells whose function of expressing wild type genes is deficient or suppressed according to any one of Claims 1 to 20, wherein a different selection marker gene from that of the gene-trap vector is linked to downstream of the promoter which is inserted in inverse direction to the endogenous gene.

22. A preparation method for cells whose function of expressing wild type genes is deficient or suppressed according to Claim 21, wherein the selection marker gene linked to downstream of the promoter which is inserted in inverse direction to the endogenous gene is puromycin resistance gene.

23. A preparation method for cells whose function of expressing wild type genes is deficient or suppressed according to any one of Claims 13 to 22, wherein antisense promoter insertion vector has a selection marker gene of the gene-trap vector, and a negative selection marker gene different from the selection marker gene linked to downstream of the promoter inserted in inverse direction to the endogenous gene, in upstream of said promoter inserted in inverse direction to the endogenous gene, and in same or inverse direction to said promoter, via a mutant or a wild type inverted repeat sequence.

24. A preparation method for cells whose function of expressing wild type genes is deficient or suppressed according to Claim 23, wherein the negative selection marker gene is thymidine kinase gene or diphtheriatoxin resistance gene that are linked to the promoter for phosphoglycerate kinase.

25. A preparation method for cells whose function of expressing wild type genes is deficient or suppressed according to any one of Claims 1 to 24, wherein the promoter inserted in inverse direction to the endogenous gene is a promoter for phosphoglycerate kinase.

26. A preparation method for cells whose function of expressing wild type genes is deficient or suppressed according to any one of Claims 2 to 25, wherein a certain position in the mutant locus is upstream region of the splice acceptor sequence.

27. A preparation method for cells whose function of expressing wild type genes is deficient or suppressed according to any one of Claims 1 to 26, **characterized in** inserting a promoter in inverse direction to the endogenous gene by expressing recombinase in the cells.

28. A preparation method for cells whose function of expressing wild type genes is deficient or suppressed according to any one of Claims 1 to 27, wherein the cells are ES cells.

29. Cells capable of transcribing antisense RNA to the trapped genes, obtained by the preparation method for cells whose function of expressing wild type genes is deficient or suppressed according to any one of Claims 1 to 28.

30. Antisense promoter insertion vector **characterized in** having a promoter capable of being inserted in inverse direction to the endogenous gene at a certain position of a mutant locus where the gene-trap vector of insertionally gene mutated trap-clone is inserted in.

31. Antisense promoter insertion vector according to Claim 30, **characterized in** having a mutant inverted repeat sequence which is a mutant of a wild type inverted repeat sequence recognized by recombinase.

32. Antisense promoter insertion vector according to Claim 31, **characterized in** having different mutant inverted repeat sequences respectively in upstream and downstream regions of the promoter which is inserted in inverse direction to the endogenous gene.

33. Antisense promoter insertion vector according to Claim 31, **characterized in** having identical mutant inverted repeat sequences respectively in upstream and downstream regions of the promoter which is inserted in inverse direction to the endogenous gene.

34. Antisense promoter insertion vector according to Claim 31, **characterized in** having a single mutant inverted repeat sequence and a single wild type inverted repeat sequence, wherein either of the sequences is in upstream region of the promoter which is inserted in inverse direction to the endogenous gene and another in downstream region of said promoter.

35. Antisense promoter insertion vector according to any one of Claims 31 to 34, wherein a mutant inverted repeat sequence is a mutant of E.Coli P1 pharge-derived wild type loxP sequence or Yeast Saccharomyces cerevisiae-derived wild type FRT sequence.

36. Antisense promoter insertion vector according to any one of Claims 31 to 35, wherein a mutant inverted repeat sequence has a mutation in an inverted repeat region.

37. Antisense promoter insertion vector according to Claim 36, wherein a mutant inverted repeat sequence, whose mutation is in the inverted repeat region, is lox71 sequence which has a base sequence shown as Seq.ID No.2 and/or lox66 sequence which has a base sequence shown as Seq.ID No.3.

38. Antisense promoter insertion vector according to any one of Claims 31 to 35, wherein the mutant inverted repeat sequence has a mutation in the spacer region which is in between the inverted repeat regions.

39. Antisense promoter insertion vector according to any one of Claims 30 to 38, wherein a different selection marker gene from that of the gene-trap vector is linked to downstream of the promoter which is inserted in inverse direction to the endogenous gene.

40. Antisense promoter insertion vector according to Claim 39, wherein the selection marker gene linked to downstream of the promoter which is inserted in inverse direction to the endogenous gene is puromycin resistance gene.

41. Antisense promoter insertion vector according to any one of Claims 30 to 40, **characterized in** having, in upstream region of the promoter which is inserted in inverse direction to the endogenous gene, said promoter and a negative selection marker gene in same or inverse direction to said promoter via a mutant or a wild type inverted repeat sequence.

42. Antisense promoter insertion vector according to Claim 41, wherein the negative selection marker gene is thymidine kinase gene or diphtheriatoxin resistance gene that are linked to the promoter for phosphoglycerate kinase.

43. Antisense promoter insertion vector according to any one of Claims 30 to 42, wherein the promoter inserted in inverse direction to the endogenous gene is a promoter for phosphoglycerate kinase.

44. A gene trap method **characterized in** using antisense promoter insertion vector according to any one of Claims 30 to 43.

45. A cell treatment method to delete or suppress expression of a specific gene in the cells, **characterized in** using antisense promoter insertion vector according to any one of Claims 30 to 43.

46. A knockout-like mouse whose gene expression is deficient or suppressed, **characterized in** being obtained by introducing antisense promoter insertion vector according to any one of Claims 30 to 43 into ES cells, and by microinjecting mice blastocysts with the ES cells.
